Europäisches Patentamt

European Patent Office

Office européen des brevets

(11) Publication number: **0 389 023 A2**

# EUROPEAN PATENT APPLICATION

(21) Application number: **90200545.3**

(22) Date of filing: **08.03.90**

(51) Int. Cl.5: **A61F 13/15**

(30) Priority: **20.03.89 US 325619**

(43) Date of publication of application:
**26.09.90 Bulletin 90/39**

(84) Designated Contracting States:
**AT BE CH DE DK ES FR GB GR IT LI LU NL SE**

(71) Applicant: **THE PROCTER & GAMBLE COMPANY**
**One Procter & Gamble Plaza**
**Cincinnati Ohio 45202(US)**

(72) Inventor: **Furio, Diane Lynn**
**7225 Overcliff Rd.**
**Cincinnati, OH 45233(US)**
Inventor: **Johnson, Theresa Louise**
**11785 Rose Ln., Apt. C**
**Cincinnati, OH 45246(US)**
Inventor: **Benson, Douglas Herrin**
**Rt. 6, Box 203, Jamison Rd.**
**West Harrison, IN47060(US)**

(74) Representative: **Suslic, Lydia et al**
**Procter & Gamble European Technical**
**Center N.V. Temselaan 100**
**B-1820 Strombeek-Bever(BE)**

(54) **Absorbent structures with odor control material.**

(57) Absorbent articles such as sanitary napkins, diapers, and the like, comprising a topsheet and an absorbent core, are provided with odor-controlling agents, especially molecular sieves, in the form of a comminuted sheet material. The sheet form simplifies the handling of the odor-controlling agents and the high-speed manufacture of the finished article. By comminuting the sheet, rather than using it in integral form, the sanitary and aesthetic advantages associated with modern topsheet materials are retained.

Fig.3

## ABSORBENT STRUCTURES WITH ODOR CONTROL

### TECHNICAL FIELD

The present invention relates to absorbent structures, such as catamenials, diapers, bandages, adult incontinence garments, and the like, which comprise a "non-stain" topsheet and an odor-control material.

### BACKGROUND OF THE INVENTION

A wide variety of absorbent structures designed not only to be efficient for the absorption of body fluids such as blood, urine, menses, and the like, but also to be sanitary and comfortable in-use are known in the literature. Disposable products of this type generally comprise some sort of fluid-permeable topsheet material, an absorbent core, and a fluid-impermeable backsheet material. Various shapes, sizes and thicknesses of such articles have been explored in an attempt to make their use more comfortable and convenient.

One of the greatest improvements in the comfort and convenience of such sanitary products has been in the development of new, "nonstaining" topsheet materials to replace the old style gauze topsheets. In general, such topsheet materials are designed to provide quick passage of the discharged fluids through the topsheet and into the absorbent core. Certain topsheets are designed to prevent rewet caused by passage of fluid back through the topsheet, and this adds considerably to consumer comfort. Moreover, by selecting topsheet materials which do not, themselves, absorb fluids, a clean, comfortable and sanitary surface is always presented to the discharge point of the body fluid.

Another aspect of sanitary products which has been under investigation for many years is that of odor control. Many body fluids have an unpleasant odor, or develop such odors when in contact with air and/or bacteria for prolonged periods. The literature is replete with references relating to odor control in products such as diapers and catamenials.

Various odor-controlling agents have been disclosed in the literature, and many of these are designed either to be sprayed onto an absorbent core in diapers and catamenial products, or to be "dusted" thereon in the form of finely-divided particles. However, dusting or spraying (e.g., from an aqueous solution) of odor-controlling agents directly onto absorbent cores is a notion that does not take into account modern high speed manufacturing equipment and materials. For example, it would be difficult to spray an aqueous solution of an antibacterial onto air-laid absorbent diaper or catamenial cores moving at a speed of 500-600 cores per minute, and then to dry the cores sufficiently to allow them to be formed into a finished article. Using dry powders, especially finely-divided powders, is also problematic, inasmuch as the powders tend to be blown or vacuumed up from the cores by the processing equipment, especially on high speed lines.

Accordingly, it would be desirable to provide an odor-controlling agent in a form which would be amenable to fabrication into absorbent structures without the aforementioned manufacturing difficulties. One method would be to combine the odor-controlling agent into a separate sheet of material and place that sheet under the topsheet so that the discharged fluid quickly comes in contact with the sheet containing the odor-controlling agent immediately after passage through the topsheet.

However, it has been determined that this apparently simple solution to the problem raises some difficulties, particularly when employed with the newer "nonstaining" topsheet materials. For one thing, use of the odor-controlling agent in the form of a continuous sheet of material undesirably adds to the stiffness of the overall product. This is particularly true in regard to modern catamenial products, which are designed to be as soft, thin, dry, pliable and comfortable as possible. Moreover, placement of a continuous sheet of material containing the odor-controlling agent immediately under the nonstain topsheet undesirably gives the appearance (if not the fact) of stain being present on the topsheet, itself. This can be particularly disturbing to users who have come to value the sanitary appearance afforded by such topsheet materials.

Moreover, placing said continuous sheet of material between the topsheet and the absorbent core can undesirably affect the acquisition of fluid by the core.

The present invention provides a means for overcoming these difficulties by providing the odor-controlling agent in the form of discontinuous pieces, strips, or the like, rather than as a single continuous sheet underlying a nonstain topsheet. Accordingly, the present invention provides not only a simplified means for manufacturing absorbent structures having odor-control properties without the difficulties associated with dustiness and drying, and fluid acquisition, noted hereinabove, but also retains the pleasant aesthetic properties afforded by the topsheet. These and other advantages associated with the present invention will be seen from the disclosure, hereinafter.

## BACKGROUND ART

The patent literature contains a considerable number of references relating to odor control in sanitary products such as diapers, bandages and catamenials. The following are illustrative.

U.S. 4,385,632 (5/31/83) by S. O. Odelhög, assigned to Landstingens Inköpscentral teaches copper odor control agents used on the surface of absorbent articles.

U.S. 3,804,094 (4/16/74) by K. Dossou, M. Gascon, G. Manoussos, assigned to L'Oreal Fr teaches a periodic acid odor control agent used on the surface of an absorbent article.

U.S. 4,525,410 (6/25/85) by Z. Hagiwara, H. Ohki, S. Hoshino, S. Nohara, S. Ida, K. Tagawa, assigned to Kanebo, Ltd. and Kanto Chemical Co., Inc. teaches zeolite particles (doped with bactericidal cations) assertedly stably held in a fibrous web by incorporating some portion of meltable fibers in the web, and applying heat; said to be useful as the "outside cover layer" in, e.g., "general sanitary goods".

Japanese J63224734-A (88.09.19) Priority 87JP-058738 (87.03.16) J63224734 ASK KK relates to a paper comprising a powder or fiber obtained by grinding sepiolite, said paper having deodorizing capacity.

Japanese J63242261-A (88.10.07) 87JP-076111 J63242261 ASK KK relates to an odor-absorbing mat with sepiolite powder, a nonwoven fabric layer, and what appears to be a sheet to which the sepiolite is attached by adhesive.

U.S. 2,690,415 (9/28/54) by F. A. Shuler teaches particles of odor-absorbing materials uniformly affixed at the interstices of a permeable web by adhesive to provide an odor absorbent medium for, e.g., catamenials. Particulate carbon, silica gel and activated alumina are noted. Shifting/displacement of the particulates is assertedly avoided and the sheet is flexible.

U.S. 3,093,546 (6/11/63) by R. L. Atkinson, teaches halogenated diphenyl methane derivatives "advantageously placed on the surface of a catamenial dressing" to "obtain prompt deodorizing activity".

Japanese J54141857 (J87019865) teaches the manufacture of powder (including zeolites) sheets by laminating the powder between a first and second sheet. Powders include activated carbon, zeolite, etc. The abstract indicates use in catamenials or deodorizing materials.

BE-815446 (Abstract) teaches sanitary towels with chlorophyll crystals or activated carbon, either in the absorbent layer, on the surface, or (per abstract) between.

ABSCENTS (odor-control molecular sieve from Union Carbide) -Use in diapers and catamenials is specifically noted in Union Carbide brochure (A. J. Gioffre 1988). The brochure indicates that UC's market research shows potential benefits in such products.

Patents relating to various topsheets, diaper and catamenial designs, and the like, are listed in the Detailed Description and Examples, hereinafter. All patent documents cited in this specification are incorporated herein by reference.

## SUMMARY OF THE INVENTION

The present invention relates to absorbent structures, comprising:

(a) a flexible, fluid-receiving front face which comprises a continuous, nonabsorbent polymeric film, said film being fluid-permeable by virtue of a multiplicity of openings or channels passing therethrough;

(b) a fluid-retaining absorbent core beneath said front face (a) comprising a major portion of fluid-retaining material, and a minor portion of odor-control material which comprises multiple pieces of comminuted (e.g., diced or shredded) sheet material containing an odor-controlling agent;

(c) a backing sheet beneath said core (b) e.g., comprising a flexible, fluid-impermeable polymeric film or flushable, biodegradable sheet; and

(d) optionally, means for retaining said structure in an appropriate position to perform its absorbency function.

In one embodiment, the odor-controlling agent used herein is an antibacterial compound. In another embodiment, the odor-controlling agent is a water-insoluble particulate odor-absorbing material, or said particulate material in combination with an antibacterial compound. Such particulate odor-controlling agents

3

include, for example, members selected from the group consisting of carbon, silica gel, zeolite (or, "molecular sieve"), activated alumina, and mixtures thereof.

In one preferred embodiment, the structures herein have a front face film which comprises a "tapered capillary" sheet (as described more fully, hereinafter). Sanitary products made from such structures include adult incontinence garments, absorbent pads (e.g., bed pads), pantiliners, catamenials and diapers, and also include bandages, foot pads, and the like.

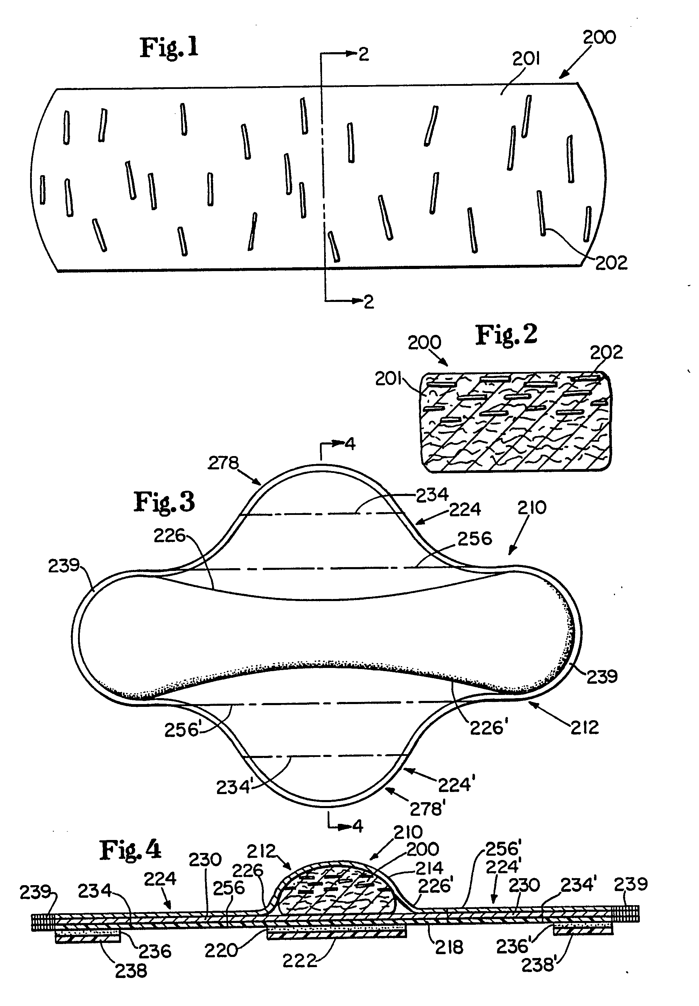

In another preferred embodiment, the structures herein have a front face film which comprises a "formed-film" sheet (as described more fully hereinafter). Sanitary products of the same type noted above can be prepared from such absorbent structures, including adult incontinence garments, absorbent pads, pantiliners, diapers and, most particularly, catamenials (sanitary napkins). A highly preferred sanitary napkin is depicted in Figure 3.

All percentages herein are by weight, unless otherwise specified.

## BRIEF DESCRIPTION OF THE DRAWINGS

FIG. 1 is a plan view of an absorbent core, or pad, 200 comprising a major portion of fluid-retaining material 201 and a minor portion of pieces of odor-control material 202 distributed discontinuously on the fluid-receiving front face of said core.

FIG. 2 is a cross sectional view of the absorbent core, or pad, of FIG. 1 taken along line 2-2 of the absorbent structure of FIG. 1. In this instance, the pieces of odor-control material are shown as being distributed preferentially towards the fluid-receiving front face of the core. Alternatively, the odor-control material can be distributed throughout the structure, or, less preferably, towards the back of the structure.

FIG. 3 is a plan view of a preferred embodiment of a sanitary napkin of the present invention.

FIG. 4 is a cross sectional view of the sanitary napkin.

## DETAILED DESCRIPTION

The articles disclosed herein can be prepared using constituents that are very well-known in current commercial practice, and reference can be made to the various patents mentioned herein and to the general sanitary products patent literature and trade catalogues for such items. Likewise, methods and apparatus for assembling disposable diapers, catamenials, bandages, and the like are known from patents and engineering literature.

While the constituents used in the assembly of catamenials, disposable diapers, and the like, are well-known, the following may be mentioned solely by way of example. It is to be understood that the present invention resides in the novel assemblage of such items, or their equivalents, into the odor-controlling absorbent structures disclosed herein, rather than in the constituents *per se*.

### I. Front-face Material

- The front-face (or, "topsheet") material used herein is a "nonstaining" hydrophobic, fluid-permeable sheet. Hydrophobic sheet materials of the type typically employed in the practice of this invention can be prepared by methods well-described in the patent literature. For example, according to the process of U.S. Patent 4,324,246, Mullane and Smith, April 13, 1982, a sample of thermoplastic material such as 0.0038 cm thick polyethylene film is heated above its softening point. (The softening point is the temperature at which the thermoplastic material can be formed or molded and is less than the melting point of the material.) The heated thermoplastic material in sheet form is then brought into contact with a heated forming screen. The forming screen is preferably an apertured wire mesh screen having the desired aperture size, pattern and configuration. A vacuum is used to draw the heated film against the forming screen, thereby forming the film into the desired pattern and having the desired hole sizes. While the vacuum is still being applied to the film, a jet of hot air is passed over the film. The hot air jet perforates the film in a pattern corresponding to the pattern and size of apertures in the forming screen.

Fluid-permeable sheets prepared in the manner of the Mullane et al patent are conveniently referred to as "formed films". The caliper of such films is important since, if the caliper is too great, liquid may accumulate in the apertures and not readily pass therethrough. For the manufacture of absorbent articles

4

such as diapers, catamenials, incontinence articles, and the like, the sheets typically have a caliper of less than about 0.075 cm, or preferably less than about 0.064 cm.

Another formed-film sheet material useful herein is the resilient, 3-dimensional web exhibiting a fiber-like appearance and tactile impression, comprising a fluid-impervious plastic material, with said web having a multiplicity of apertures, the apertures being defined by a multiplicity of intersecting fiber-like elements, all as disclosed in U.S. Patent 4,342,314, Radel and Thompson, August 3, 1982. The Radel and Thompson sheet materials can be prepared using hydrophobic plastics such as polyethylene, polypropylene, PVC, and the like, and are well-known for use in absorbent products such as catamenials, and the like.

Yet another type of sheet material useful herein is described in U.S. Patent 3,929,135, Thompson, December 30, 1975, and consists of hydrophobic polymer films having holes which are in the form of tapered capillaries. These "tapered capillary" sheets are also known for use in absorbent articles, including adult incontinence articles. They may be prepared from various hydrophobic polymers, as mentioned hereinabove; typically, low density polyethylene having thickness of from 0.0025 to 0.0051 cm is employed.

Reference to U.S. Patent 3,929,135 can be made in order to further visualize tapered capillary sheets. In use, the apices of the capillaries in such tapered capillary topsheets are in contact with the underlying absorbent core material. Generally, tapered capillaries are in the form of a frustrum of a conical surface, but it is to be understood that any generally tapered structure, such as a frustrum of a pyramid or the like with a triangular, square, or polygonal base, is within the term "tapered capillary"; circular tapered capillaries, however, are used in this description for convenience. It is also to be understood that the tapered capillaries can be asymmetric (i.e., the angle of taper on one side can be different from that on another side) and that the angle of taper can change continuously (i.e., be curved) over the distance from base to apex. In the latter case, the angle of taper is defined as the angle of the tangent to the side of the capillary at its point of minimum apex opening dimension. The angle of taper suitable for use in topsheets according to the practice of this invention is from about 10$^\circ$ to about 60$^\circ$.

Base opening dimension of the capillaries is defined as the maximum open measurement in the plane of topsheet at said tapered capillary. Apex opening dimension is defined as the maximum open measurement in the apex of said tapered capillary, which apex is remote from the plane of the topsheet. When the tapered capillary is in the form of a frustrum of a conical surface, the base and apex opening dimensions are, respectively, the base diameter and the apex diameter. Base diameter and apex diameter are hereinafter used interchangeably with, respectively, base opening dimension and apex opening dimension.

The tapered capillary apex diameter is a diameter which will allow liquid to readily pass from the surface of the topsheet to the underlying absorbent core. The apex diameter is from about 0.004 to about 0.100 inch (0.010 to 0.254 centimeter), preferably from about 0.005 to about 0.020 inch (0.013 to 0.051 centimeter).

The tapered capillary base diameter is selected to satisfy two criteria. The first of these is the subjective feel of the surface of the topsheet which contacts the skin of the user. It has been discovered that polyethylene can be made to exhibit pleasing, clothlike, non-waxy attributes when the base diameter is within the range from about 0.006 to about 0.250 inch (0.015 to 0.635 centimeter). Preferably, the base diameter should be within the range of from about 0.030 to about 0.060 inch (0.076 to 0.152 centimeter). The second criterion is that the capillary base diameter be small enough to allow an expected liquid droplet to bridge across at least one capillary. This criterion is satisfied by the above dimensions for disposable diapers and sanitary items.

The height of the tapered capillary is defined as the distance between the outermost surface of the topsheet (i.e., that surface which normally contacts the skin of the user) and the apex of the tapered capillary. This height, of course, depends upon apex diameter, base diameter, and angle of taper which have been selected as hereinbefore described. The height of the tapered capillary should provide a structure with a minimum tendency to collapse in use. The characteristics of the material of construction of the topsheet in large measure determine suitable ranges for the height. When the topsheet is low density polyethylene of from 0.001 to 0.002 inch (0.003 to 0.005 cm) thickness and apex diameter and base diameter are in the preferred range, and angle of taper $\alpha$ is in its critical range, the height of the tapered capillary can be from about 0.003 to about 0.159 inch (0.008 to 0.404 centimeter).

A state of relative dryness on the surface of the topsheet implies that most of the liquid which contacts the topsheet is transferred through it to the absorbent element. This in turn implies that each isolated droplet of fluid in contact with the topsheet must be in contact with the base diameter of a tapered capillary. This state of affairs can best be achieved if the land area (the area of the topsheet that exists between the bases of the tapered capillaries) is maintained at a minimum. The minimum limiting value is the case where conical tapered capillaries or pyramidal tapered capillaries are provided in close packed array (where the periphery of the base of each capillary is in contact on all sides with the periphery of the base of adjacent capillaries). The preferred arrangement of minimum land area tends to insure that an individual droplet will contact at least one tapered capillary. A preferred arrangement in disposable diapers is where the tapered capillaries as hereinbefore described are in ordered arrangement with from about 30 to about 1500 tapered capillaries per square inch of topsheet (5 to 231 per square centimeter).

Tapered capillary sheets can be manufactured in any of several ways well known in the art. One particularly suitable method is to provide a heated mold with male elements of the shape and arrangement of the desired tapered capillaries (hereinafter a pin mold). Each male element is secured in such a fashion that its apex extends away from the base of the pin mold. A portion of sheet material is brought into contact with the heated pin mold between the mold and a resilient backing plate. Pressure is applied to the combination of mold, sheet and resilient back plate and tapered capillaries are formed in the sheet to make the tapered capillary topsheet. An alternate way of constructing the topsheet is to subject a portion of liquid-impervious material to vacuum forming over an appropriate mold. After forming tapered capillary sheets in one of the aforementioned ways, it may be necessary to physically remove material from the apices of the capillaries so as to insure that the apex diameters are the desired value. Such removal of material can be accomplished by, for example, subjecting the apices to controlled abrasion or by heating the formed topsheet so as to melt open the apices. See, also, U.S. Patent 4,629,643, Curro and Linman, December 16, 1986, for a microapertured polymeric film with improved tactile impression, which can also be used in the practice of this invention.

A highly-preferred fluid-permeable formed-film sheet material which can be employed in the practice of this invention is disclosed in U.S. Patent 4,463,045, Ahr et al, July 31, 1984, and reference can be made to that patent to further assist visualization of the Ahr et al structures.

In general terms, the sheets provided by U.S. Patent 4,463,045 are designed not only to provide a desirable cloth-like tactile impression, but also to substantially eliminate surface gloss. Thus, sheets made of plastic do not have an undesirably shiny, "plasticky" appearance.

Such highly-preferred sheet materials can be succinctly described as being a macroscopically expanded three-dimensional plastic "web" having at least one visible surface which appears substantially nonglossy when exposed to light, substantially all of said visible surface exhibiting a regularly spaced, microscopic pattern of discrete surface aberrations, each of said surface aberrations having its amplitude oriented perpendicular to the surface in which said surface aberration originates, each of said surface aberrations having a maximum dimension of less than about 6 mils, as measured in a plane oriented substantially perpendicular to its amplitude, whereby said surface aberrations are not discernible to the normal naked eye when the perpendicular distance between the viewer's eye and the plane of said web is at least about 12 inches, each of said surface aberrations also being free of planar areas which are large enough to inscribe a 4 mil diameter circle and so spaced relative to all adjacent surface aberrations that the maximum diameter of any circle which can be inscribed on any planar surface intermediate said surface aberration and said adjacent surface aberrations on any portion of said visible surface is less than about 4 mils, whereby any light incident upon any portion of said visible surface is diffusely reflected into a multiplicity of directions by said surface aberrations so that said visible surface appears substantially nonglossy.

The 045 sheet materials can have at least a portion of said surface aberrations comprising protuberances projecting generally outwardly from the surface, and can have at least a portion of said surface aberrations comprising depressions projecting generally inwardly from the surface of said web.

The manufacture of these preferred sheets can be achieved by use of a forming screen or structure, as generally noted hereinabove, which provides said surface aberrations by virtue of "knuckles" on the support member. (The preparation of such sheets is described in great detail in U.S. Patent 4,463,045, and their method of preparation forms no part of this invention.) In general, the resulting surface aberrations correspond to the knuckles of a woven mesh support structure which directly contacts the visible surface of said plastic sheet during production thereof.

In a preferred manufacturing method, the woven mesh support structure which directly contacts the visible surface of said sheet is comprised of filaments having a diameter between about one and about two mils and a mesh count between about 160 filaments per lineal inch (2.54 cms) by 160 filaments per lineal inch (2.54 cms) and about 400 filaments per lineal inch (2.54 cms) by 400 filaments per lineal inch (2.54 cms).

Preferred sheets herein are those wherein said surface aberrations have an average amplitude of at least about 0.2 mils, more preferably at least about 0.3 mils. Most preferably, sheets having an amplitude of each of said surface aberrations, as measured perpendicular to the surface in which said surface aberration originates, within the range of about ± 20%, desirably ± 10%, of the average value of the amplitude for all adjacent surface aberrations are used.

"One-way" sheets whose back faces are treated with hydrophilic latex are described in U.S. Patent 4,735,843, Noda, April 5, 1988, and these can also be employed herein.

In addition to the sophisticated apertured materials mentioned hereinabove, the practice of the present invention may also be undertaken with hydrophobic sheet materials having simple holes punched therethrough.

It will be understood from the foregoing that the "sheet" or "film" materials used in the practice of this invention are substantially different from fibrous nonwoven materials, which are characterized by a large number of fibers which overlap each other throughout the thickness of the material. Moreover, the sheets used in the practice of this invention are made from materials (preferably, hydrophobic thermoplastic polymeric materials) which provide a clean-appearing, stain-resistant or "non-staining" surface, in use.

The most preferred way to check stain appearance is by *in vivo* testing with users, using visual standards and standard statistical techniques. If desired, an *in vitro* test can be devised. The following *in vitro* test protocol is only by way of illustration, and modifications can be made, depending on the desires of the formulator. See U.S. Patent 4,324,246, above.

A synthetic menstrual fluid is prepared by adding approximately 15 grams of the pulp from oranges to 100 milliliters of a 9% sodium chloride solution and blending for about 1 minute. Four grams of crystalline bovine albumin are dissolved in the sodium chloride solution and 33 milliliters of whole blood together with 25 grams of egg white are added. The mixture is stirred until it is uniform. While any synthetic menstrual fluid can be used, it is important that the fibrous and mucosal components of menses be simulated.

A grading scale for determining the cleanliness rating of various stain-resistant sheets (e.g., sanitary napkin topsheets) is prepared. A substrate which will retain all of the synthetic menstrual fluid placed on it is selected and several substrate samples prepared. A spun bonded polyester nonwoven web having a basis weight of 0.5 ounces per square yard (17 $g/m^2$) manufactured by E. I. DuPont deNemours of Wilmington, Delaware and marketed under the tradename T-310 can be used. Varying amounts of the synthetic menstrual fluid are applied to a 2.5 x 7.6 cm rectangular portion of each substrate sample. To provide a typical grading scale, multiple substrate samples can be treated with 0, 0.1, 0.25, 0.50, 1.0, 1.5, 2.0, and 4.0 milliliters of synthetic menstrual fluid. No absorbent core is provided beneath the substrate sample. Therefore, all the menstrual fluid placed on the substrate sample remains on the sample. The menstrual fluid is allowed to dry and each substrate sample is assigned a value ranging from 0 to 7, respectively. Thus, the sample treated with no synthetic menstrual fluid is assigned a value of zero and indicates a clean substrate while the sample treated with 4.0 ml of synthetic menstrual fluid is assigned a value of 7 and indicates a heavily soiled substrate.

The topsheet to be tested for stain qualities is placed over, and in contact with, an absorbent core and 4.0 mls of synthetic menstrual fluid are spread over a 2.5 x 7.6 cm rectangle of the topsheet. After 60 seconds, the topsheet material is removed from the absorbent core and allowed to dry. To facilitate the even distribution of the synthetic menstrual fluid over the test rectangle, a small amount of synthetic menstrual fluid (0.2-0.3 ml) can be spread over the rectangle before the topsheet material is placed over the absorbent core.

The test topsheet material is then visually compared with the grading scale to determine its cleanliness, and can be assigned a cleanliness rating.


II. Absorbent Material

- The absorbent material used herein can comprise any of the well-known fluid (e.g., blood-, urine- or menses-) absorbent materials such as cotton cloth, cellulose fibers, "super absorbent" polymers, and mixtures thereof.

As is well-known from recent commercial practice, fluid-retaining cores comprising absorbent gelling materials (sometimes referred to as "super-sorbers") are becoming broadly accepted by consumers. Such materials form hydrogels on contact with water (e.g., with urine, blood, and the like). One highly preferred type of hydrogel-forming, absorbent gelling material is based on polyacids, especially polyacrylic acid. Hydrogel-forming polymeric materials of this type are those which, upon contact with fluids (i.e., liquids) such as water or body fluids, imbibe such fluids and thereby form hydrogels. In this manner, fluid discharged into the absorbent structures herein can be acquired and held. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer material; and (4) polyvalent metal compounds which can form ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the absorbent gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization." Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issued March 31, 1987, Reissue 32,649. The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials hereinbefore described can be incorporated into the cores of the absorbent articles of the present invention in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reasons of industrial hygiene, average particle sizes smaller than about 30 microns are less desirable. Particles having a smallest dimension larger than about 2 mm may also cause a feeling of grittiness in the absorbent article, which is undesirable from a consumer aesthetics standpoint. Furthermore, rate of fluid absorption can be affected by particle size. Larger particles have very much reduced rates of absorption. Preferred for use herein are absorbent gelling material particles substantially all of which have a particle size of from about 30 microns to about 2 mm. "Particle Size" as used herein means the weighted average of the smallest dimension of the individual particles.

The amount of absorbent gelling material particles used in absorbent cores will depend upon the degree of absorbent capacity desired, and will generally comprise from about 2% to 50% by weight of the absorbent core, more typically from about 5% to 20% by weight of the absorbent core.

When absorbent gelling material particles are to be used in the cores of the absorbent articles herein, such cores can be prepared by any process or technique which provides a web comprising a combination of the fibers and the gelling material particles. For example, web cores can be formed by air-laying a substantially dry mixture of hydrophilic fibers and absorbent gelling material particles and, if desired or necessary, by densifying the resulting web. Such a procedure is described more fully in Weisman and Goldman; U.S. Patent 4,610,678; Issued September 9, 1986. As indicated in this U.S. Patent 4,610,678, the air-laid webs formed by such a procedure will preferably comprise substantially unbonded fibers and will preferably have a moisture content of 10% or less.

The density of the absorbent cores which comprise webs of hydrophilic fibers and absorbent gelling material particles can be of importance in determining the absorbent properties of the cores and of the absorbent articles in which such cores are employed. The density of such absorbent cores herein will preferably be in the range of from about 0.06 to about 0.3 $g/cm^3$, and more preferably within the range of from about 0.09 to about 0.22 $g/cm^3$. Typically the basis weight of the absorbent cores herein can range from about 0.02 to 0.12 $g/cm^2$.

Density values for cores of this type can be calculated from basis weight and caliper. Caliper is measured under a confining pressure of 0.137 psi (0.94 kPa). Density and basis weight values include the weight of the absorbent gelling materials and the odor-control material. Density of the cores herein need not be uniform throughout the core. Within the density ranges hereinbefore set forth, the cores can contain regions or zones of relatively higher or relatively lower density.

## III. Backing Sheet

- The backing sheet is conventional, and can comprise a fluid-impervious polymer sheet, for example polyethylene or polypropylene, that is thin enough to be flexible. A polyethylene sheet 0.001-0.5 mm thick is typical. Flushable or biodegradable backing sheets can also be used, e.g., with pantiliner devices herein.

## IV. Odor-Controlling Agent

- The odor-controlling agent employed in the practice of this invention can be a water-soluble antibacterial compound. Such materials include, for example, halogenated phenylene compounds (U.S. Patent 3,093,546), periodic acid (U.S. Patent 3,804,094), various copper compounds, especially copper acetate (U.S. Patent 4,385,632), various quaternary ammonium salts, which are well-known for their antibacterial properties, e.g., cetyl pyridinium chloride, and the like. Alternatively, antibacterial compounds can be used conjointly with various particulate materials which, in use in the presence of moisture, release the antibacterial agent. Zeolite materials, such as zeolites which are bactericidal by virtue of having absorbed therein and thereon various bactericidal cations such as copper, silver and zinc, can be advantageously used in the practice of this invention (U.S. Patent 4,525,410). In a preferred mode, the odor-controlling agent is a water-insoluble particulate odor-absorbing material such as chlorophyll particles, activated carbon granules (8-140 mesh), ion exchange resin (Japanese 87019865), activated alumina, and absorbent zeolite materials, including the well-known "molecular sieve" zeolites of the type A and X zeolites (generally in the 1-10 micron particle size range) and the zeolite materials marketed under the trade name ABSCENTS by the Union Carbide Corporation and UOP, and which are typically available as a white powder in the 3-5 micron particle size range (see: ABSCENTS, A New Approach for Odor Control by A. J. Gioffre, copyright 1988 by the Union Carbide Corporation).

The use of preferred zeolite (or, "molecular sieves") of the ABSCENTS type to control odors is fully described in U.S. Patent 4,795,482, January 3, 1989, to Gioffre and Marcus. In general, these preferred molecular sieve odor-controlling agents appear to function by entrapping odoriferous substances within their molecular lattice structures. Whatever their mode of action, these odor-controlling agents can be characterized by their physical parameters, as follows. These agents are reported by Gioffre and Marcus to be crystalline siliceous molecular sieves in which at least about 90, and preferably at least about 95, percent of the framework tetrahedral oxide units are $SiO_2$ tetrahedra and which have a sorptive capacity for water at 25°C and 4.6 torr of less than 10 weight percent. In the case of aluminosilicate molecular sieves, those preferred in the practice of this invention have a framework $SiO_2/Al_2O_3$ molar ratio of from about 35 to infinity, and preferably from 200 to 500. Such siliceous molecular sieves have a pore diameter of at least 5.5 Angstroms, preferably at least 6.2 Angstroms. Preferably the adsorption capacity for water vapor at 25°C and a water vapor pressure (p/p₀) of 4.6 torr is less than 6 weight percent. The efficacy of these preferred molecular sieves employed in the practice of the present invention is not dependent upon the presence of the water of hydration in the internal cavities of the microporous structure as a result of their hydrothermal formation. In fact, at least a major proportion, usually substantially all, of this original water of hydration is removed in the process of removing any pore-blocking templating agent which may be present in the adsorbent. Calcination effectively removes any organic moieties. Also, water washing or washing with a caustic or dilute mineral acid solution is advantageously utilized to remove extraneous synthesis reactants from the pore system. Lowering of the alkali metal content, particularly the nonzeolitic, i.e., occluded alkali metal compounds can also be beneficial. These procedures also serve to remove the original water of hydration.

As further disclosed by Gioffre and Marcus, such siliceous molecular sieves include the microporous crystalline aluminosilicates, i.e., the zeolitic molecular sieves as well as the so-called silica polymorphs. With respect to the latter compositions, their crystal lattices are ideally formed entirely of $SiO_2$ tetrahedral units, but the as-synthesized forms commonly contain at least trace amounts of aluminum derived from aluminum impurities in the synthesis reagents. The aluminosilicate molecular sieves comprise the large class of well-known crystalline zeolites. These high-silica molecular sieves are either commercially available or are prepared by methods well-known in the art, involving direct hydrothermal synthesis or involving certain types of crystal lattice dealuminations. A comprehensive review article by E. M. Flanigen concerning both "high" Si/Al zeolites and silica molecular sieves is published in "Proc. 5th Int. Conf. Zeolites, Naples, 1980", L. V. C. Rees, ed., Heyden, London, pp. 760-780.

With respect to the foregoing odor-controlling agents, it is important that their pore system be open so that the internal cavities of the crystals be accessible to the odor molecules. In the case of the aluminosilicates or silica polymorphs produced using large organic templating ions such as tetraalkylammonium ions, it is necessary to remove charge balancing organic ions and any occluded templating material in order to permit adsorption of the odor molecules. In such a removal process and also in the removal of inorganic debris, the original water of hydration is also removed. Upon exposure to the atmosphere, a portion of the water of hydration is reacquired, but this does not affect the characteristics of the molecular sieves which are preferred for the practice of the present invention, i.e., the molecular sieves can be employed in either a hydrated or dehydrated state, but, in general, the dehydrated state is preferred. In the case of most of the dealumination procedures referred to above, the original water of dehydration is also removed, and can similarly be replaced, if desired, for the practice of the invention.

More specifically, Gioffre and Marcus disclose that the class of medium to large pore siliceous molecular sieves, from which the original, as-synthesized water of hydration has been substantially removed, and which have a capacity for adsorbed water of not greater than 10, and preferably not greater than 6, weight percent when measured at 25°C and a water vapor pressure (p/p₀) of 4.6 torr, function in an extraordinary manner with respect to odor elimination. Many of the synthetic zeolites prepared using organic templating agents are readily prepared in a highly siliceous form - some even from reaction mixtures which have no intentionally added aluminum. These zeolites are markedly organophilic and include ZSM-5 (U.S. Patent 3,702,886); ZSM-11 (U.S. Patent 3,709,979); ZSM-35 (U.S. Patent 4,016,245); ZSM-23 (U.S. Patent 4,076,842); and ZSM-38 (U.S. Patent 4,046,859) to name only a few. It has been found that the silica molecular sieves known as silicalite and F-silicalite are particularly suitable for use in the present invention and are thus preferred. These materials are disclosed in U.S. Patents 4,061,724 and 4,073,865, respectively. To the extent the aforesaid siliceous sieves are synthesized to have $SiO_2/Al_2O_3$ ratios greater than 35, they are frequently suitable for use in the present articles without any additional treatment to increase their degree of hydrophobicity. Molecular sieves which cannot be directly synthesized to have both sufficiently high Si/Al and/or degree of hydrophobicity ratios can be subjected to dealumination techniques, fluorine treatments and the like, which result in organophilic zeolite products. High-temperature steaming procedures for treating zeolite Y which result in hydrophobic product forms are reported by P. K. Maher et al, "Molecular Sieve Zeolites", Advan. Chem. Ser. 101, American Chemical Society, Washington, D.C., 1971, p. 266. A more recently reported procedure applicable to zeolite species generally, involves dealumination and the substitution of silicon into the dealuminated lattice site. This process is disclosed in U.S. Patent 4,503,023 issued March 5, 1985 to Skeels et al. Halogen or halide compound treatments for zeolites to increase their hydrophobicity are disclosed in U.S. Patents 4,569,833 and 4,297,335. Steam-treated zeolite Y, prepared per U.S. Patent 4,331,694, and denominated "LZ-10", is a particularly useful odor-controlling agent.

Various modified zeolite-type materials, such as the manganese-aluminum-phosphorus-silicon-oxide molecular sieves described in U.S. Patent 4,793,833, Lok et al, assigned to UOP, can be used herein. See also U.S. Patents 4,604,110; 4,437,429; and 4,648,977, for other zeolitic odor-controlling compositions.

## Optional Retaining Means

- The absorbent structures herein can optionally, but preferably, be provided with means to hold them in place on or near the user's body to allow the structures to perform their intended function. For example, diapers and incontinence garments can be provided with well-known commercially-available tape fasteners. Sanitary napkins can be provided with glue stripes facing outward on their backsheet in well-known fashion. Various pins, clips and fasteners of well-known types can optionally be employed.

## Odor-Control Material

- It is to be understood that the odor-control material employed in the practice of this invention is not, simply, the odor-controlling agent, *per se*, added to the absorbent structure. Rather, the odor-controlling agent is formed into strips, particles, or the like. In a typical method, this forming process involves combining the odor-controlling agent with a suitable carrier material, forming said agent/carrier mixture into a sheet, and then cutting, slicing or otherwise comminuting said sheet into the desired size range. The preparation of an odor-control material in this fashion is illustrated in Example I. Standard papermaking equipment is used.

## EXAMPLE I

Southern Softwood Kraft (SSK) wood pulp is introduced into a papermaking apparatus (Formar, Noble & Wood Company) and passed onto the forming screen of the apparatus. While in the damp state on the forming screen, a 10% (wt.) aqueous suspension of zeolite-type molecular sieve powder (ABSCENTS; Union Carbide Corporation; 3-5 micron average particle size) is sprayed onto the damp pulp at a rate sufficient to give approximately 60% wt. (range 50-62%) ABSCENTS loading on/in the resulting sheet (dry weight basis). The pulp/ABSCENTS mix is thereafter dried (165-179°C) to provide a sheet/ABSCENTS composite which has, generally, the appearance and consistency of commercial paper toweling (caliper ca. 12 mils; basis weight range 42-55 lbs/3000 ft$^2$) (ca. 19-25 kg/280 m$^2$).

The sheet prepared in the foregoing manner is thereafter comminuted to provide confetti-like pieces 3/32 in. x 5/8 in. (ca. 0.238 cm x 1.59 cm) by passing the above-prepared sheet through a commercial paper shredding machine (Ideal Paper Shredder - Trade Mark DESTROYIT, 4001 Cross-Cut). The resulting pieces are a preferred odor-control material employed in the practice of the present invention.

The "confetti" pieces of odor-control material are then introduced into a fluid-retaining absorbent core which comprises a major portion of fluid-retaining material and a minor proportion of said odor-control material. 0.8 g of ABSCENTS-containing odor-control material (60% loading of ABSCENTS) prepared in the foregoing manner is airlaid onto and into the surface and uppermost layers of an airlaid absorbent core comprising 8.5-9.5 grams of fluid-retaining SSK air-felt. The core is formed as a pad (surface area 18.14 in$^2$; 117 cm$^2$) which is suitable for use in a sanitary napkin.

## EXAMPLE II

A disposable baby diaper using the odor-control material of Example I is prepared as follows. The dimensions listed are for a diaper intended for use with a child in the 6 to 10 kilogram size range. These dimensions can be modified proportionately for different size children, or for adult incontinence briefs, according to standard practice.

1. Backsheet: 0.025-0.070 mm polyethylene; width at top and bottom 33 cm; notched inwardly on both sides to a width-at-center of 28.5 cm; length 50.2 cm.

2. Topsheet: tapered capillary polyethylene topsheet, per U.S. Patent 3,929,135, described hereinabove; width at top and bottom 33 cm; notched inwardly on both sides to a width-at-center of 28.5 cm; length 50.2 cm.

3. Absorbent core: air-laid wood pulp fibers, Taber stiffness range 7-9.5, 8.4 mm thick, calendered; width at top and bottom 28.6 cm; notched inwardly at both sides to a width-at-center of 10.2 cm; length 44.5 cm. Odor-control material of Example I (0.8 g) air-laid uniformly, but discontinuously, onto the fluid-receiving surface of core.

4. Elastic leg bands: four individual rubber strips (2 per side); width 4.77 mm; length 370 mm; thickness 0.178 mm (all the foregoing dimensions being in the relaxed state).

The diaper of Example II is prepared in standard fashion by positioning the core-plus-odor control material covered with the topsheet on the backsheet and gluing.

The elastic bands (designated "inner" and "outer", corresponding to the bands closest to, and farthest from, the core, respectively) are stretched to ca. 50.2 cm and positioned between the topsheet/backsheet along each longitudinal side (2 bands per side) of the core. The inner bands along each side are positioned ca. 55 mm from the narrowest width of the core (measured from the inner edge of the elastic band). This provides a spacing element along each side of the diaper comprising the flexible topsheet/backsheet material between the inner elastic and the curved edge of the core. The inner bands are glued down along their length in the stretched state. The outer bands are positioned ca 13 mm from the inner bands, and are glued down along their length in the stretched state. Since the topsheet/backsheet assembly is flexible, the glued-down bands contract to elasticize the sides of the diaper.

## EXAMPLE III

A catamenial product in the form of a sanitary napkin having one or, preferably, two flaps extending outward from its absorbent core is depicted in Figs. 3 and 4. A more detailed description of articles of this type appears in U.S. Patent 4,687,478, Van Tillburg, August 18, 1987.

As shown, sanitary napkin 210 comprises an absorbent means represented by central absorbent means 212 and two flaps 224 and 224'. (In the discussion that follows, unless otherwise noted, the sanitary napkins will have two flaps. While it is not necessary that the flaps be identical, or, more properly, mirror images one of the other, they preferably are. Thus the description of the first will be a description of the second. Discussion of the second will, therefore, be omitted for clarity of exposition. Corresponding elements are indicated in the drawings by reference numerals and primed reference numerals.)

Flap 224 is associated with central absorbent means 212 along nonlinear line of juncture 226. As used in the context of the present example, the term "nonlinear" refers to any of various curved, as opposed to straight, lines.

Flap 224 has distal edge 278 which is remote from line of juncture 226. In the embodiment illustrated in FIG. 3, line of juncture 226 is concave relative to distal edge 278. That is to say, line of juncture 226 curves away from distal edge 278. In this embodiment, nonlinear lines of juncture 226 and 226' and seam 239 define an absorbent means (central absorbent pad 212) which is narrower in its central region than at its ends. A sanitary napkin narrower in the center than at the ends is generally perceived by the user as more comfortable than a sanitary napkin of uniform width.

Flap 224 is provided with first axis of flexibility 256 and second axis of flexibility 234.

FIG. 4, a cross sectional view of sanitary napkin 210 taken along line 2-2, illustrates generally the preferred construction for sanitary napkin 210. As shown in FIG. 4, central absorbent means 212 comprises absorbent core 200, shown curved, which corresponds to the core prepared in Example I, above. Central absorbent means 212 and flap 224 both comprise topsheet 214. Backsheet 218 is disposed on the side of absorbent core 200 and flap 224 opposite that of topsheet 214. In effect, topsheet 214 forms one surface of flap 224 while backsheet 218 forms the other surface; topsheet 214 also forms one surface of central absorbent means 212, backsheet 218 the other.

In the embodiment illustrated, flap absorbent core 230 is interposed between topsheet 214 and backsheet 218. For simplicity of construction, flap absorbent core 230 extends throughout napkin 210 and is interposed between absorbent core 200 and backsheet 218. While this is a preferred embodiment primarily for reasons of construction, it is not necessary that flap absorbent core 230 be interposed between absorbent core 200 and backsheet 218.

Topsheet 214 and backsheet 218 are joined at seam 239 around the entire periphery of sanitary napkin 210. The purpose of this seam is to unite the various elements of the sanitary napkin into a whole. Topsheet 214 is secured to flap absorbent core 230 and backsheet 218 along nonlinear lines of juncture 226 and 226' by attachment means not illustrated in FIG. 4.

Illustrated in FIG. 4 are the adhesive attachment means at central pad adhesive 220 and flap adhesive 236 which are covered by, respectively, central pad release liner 222 and flap release liner 238. These adhesive attachment means are adapted to secure sanitary napkin 210 within the crotch region of an undergarment.

In this preferred embodiment, topsheet 214 is the non-glossy, formed-film, liquid permeable sheet of U.S. Patent 4,463,045, cited hereinabove. When sanitary napkin 210 is in use, Topsheet 214 is in close proximity to the skin of the user. Topsheet 214 is compliant, soft feeling and nonirritating to the user's skin.

In preferred embodiments, the inner surface of topsheet 214 is secured in contacting relation to absorbent core 200. This contacting relationship results in liquid penetrating topsheet 214 faster than if it were not in contact with absorbent core 200. Topsheet 214 can be maintained in contact with absorbent core 200 by applying adhesive, preferably in spaced, limited areas, to the inner surface of the topsheet 214. Examples of suitable adhesives used for this purpose include the acrylic emulsion E-1833BT manufactured by Rohm and Haas Company of Philadelphia, PA and the acrylic emulsion WB3805 manufactured by H. B. Fuller Company of St. Paul, MN. The adhesives can be applied by the same methods as the surfactant is applied to the outer surface of topsheet 214.

Referring to FIG. 4, it can be seen that absorbent core 200 is positioned between topsheet 214 and backsheet 218. Absorbent core 200 provides the means for absorbing menstrual fluid, and, when prepared in the manner of Example I, for controlling odor.

Backsheet 218 is impervious to liquids and, thus, prevents menstrual fluid which may be expressed from absorbent core 200 from soiling the clothing of the user.

As can be seen from the foregoing, the present invention also provides absorbent pads, or the like, comprising a major portion of fluid-retaining material and a minor, but odor-controlling, portion of odor-control material, said odor-control material comprising multiple pieces of comminuted sheet material containing an odor-controlling amount of an odor-controlling agent.

Typically, such pads will comprise from about 1% to about 25%, more generally 3% to 20%, of the odor-control material, the balance comprising the fluid-retaining material. These proportions can vary, somewhat, depending on the nature and intensity of the odors being controlled, the specific odor-controlling agent being used, and the loading level of the odor-controlling agent. Olfactory tests can routinely be used to assist the formulator in determining the amounts to be used. For the preferred ABSCENTS-type odor-controlling agent, it is preferred to provide from about 0.5 g. to about 3 g. ABSCENTS in a typical sanitary napkin pad weighing from about 4 g. to about 12 g. Since ABSCENTS can be loaded onto a carrier substrate (sheet material) at levels as high as 50-60% to provide an odor-control material, the amount of odor-control material to be used can be readily determined. However, in calculating the amount used, consideration should be given to losses of odor-controlling agent (especially solid odor-controlling agents) that can occur when said sheet materials are comminuted. Depending on the vigor and degree of comminution, experience shows that as much as 50% losses can occur. In any event, this can be taken into account when fashioning said pads. The following illustrates the formation of pads, according to the practice of this invention.

EXAMPLE IV

Following the procedure of Example I, strips (1.9 cm x 0.6 cm) of odor-control material comprising odor-controlling agents loaded on paper are prepared, as follows.

| Odor-Controlling Agent | Loading (% wt.) |
|---|---|
| (A) Cupric Acetate | 2% |
| (B) Copper-Zeolite A (1-10 microns) | 40% |
| (C) Zinc-Zeolite A (1-10 microns) | 35% |
| (D) Periodic Acid | 0.5% |
| (E) Silica Gel | 30% |
| (F) Alumina (1-5 micron) | 20% |
| (G) Activated Carbon | 12% |

The above-described odor-controlling materials A-G are each formed into absorbent pads in the manner of Example I.

## EXAMPLE V

A highly absorbent, thin pad which can be used in sanitary napkins, disposable diapers, and the like, is prepared by dry-mixing the following components.

| Component | Wt. Ratio in Mix |
|---|---|
| Fibers* | 85 |
| Absorbent Gelling Material ** | 15 |
| Odor-Control Material*** | 2 |

*Southern softwood slash pine.
**SANNWET IM 1000; Sanyo Co., Japan; acrylate grafted starch, average particle size 250 micron range.
***Confetti pieces, per Example I; 60% ABSCENTS loading.

Absorbent pads are prepared by air-laying the above mixture. Pad basis weight 390 g/m$^2$. A hydraulic press can be used to compress the pads to a thickness, typically in the 1-5 mm range. The pads are useful as the absorbent core of thin, comfortable sanitary napkins, diapers, bed pads, and the like.

## EXAMPLE VI

The pad of Example V is prepared, but with the SANWET being replaced by "low-extractables" polyacrylate absorbent gelling material, as disclosed by Brandt et al, U.S. Reissue Patent 32,649, cited hereinabove. ABSCENTS loading 1.4 g.

## EXAMPLE VII

A typical absorbent pad* suitable for use in a sanitary napkin is characterized by the following parameters.

14

| Caliper** 0.69 in. (1.75 cm)<br>Basis weight 713 g/m² <br>Dimension 7.5 in x 2.5 in (19.5 cm x 6.35 cm) | Mean Core Wt. (Total)<br>8.36 g. - Comprising:<br>i) ABSCENTS<br>ii) Fluff<br>iii) Tissue (to carry ABSCENTS; shredded)<br>to | <br><br>1.27<br>5.60<br>1.49<br>8.36 g |
| --- | --- | --- |
| *Optionally, with surfactant spray-on | | |

**Measured at 0.13 psi (9.14 g/cm²)

## EXAMPLE VIII

A pantiliner suitable for use between menstrual periods, and which can be disposed of in a toilet (i.e., "flushable") comprises a pad according to Example V, interposed between the topsheet of U.S. Patent 4,463,045 and a fibrous, nonwoven, flushable backsheet.

## EXAMPLE IX

The absorbent core of Example I is modified by adding 500 ppm cetyl pyridinium chloride to the SSK pulp (spray-on). In use as a sanitary napkin, the CPC provides odor control benefits in conjunction with the ABSCENTS odor-controlling agent.

## Claims

1. An absorbent structure, comprising:
(a) a flexible, fluid-receiving front face;
(b) a fluid-retaining absorbent core beneath said front face (a), comprising a major portion of fluid-retaining material and a minor portion of odor-control material;
(c) a backing sheet beneath said core (b); and
(d) optionally, means for retaining said structure in an appropriate position to perform its absorbency function, said structure being characterized in that said front-face (a) comprises a continuous, nonabsorbent polymeric film, said film being fluid-permeable by virtue of a multiplicity of openings or channels passing therethrough; and further characterized in that said odor-control material comprises multiple pieces of comminuted sheet material containing an odor-controlling agent.

2. A structure according to Claim 1, wherein the odor-controlling agent is an antibacterial compound.

3. A structure according to Claim 1 wherein the odor-controlling agent is a water-insoluble particulate odor-absorbing material, or said particulate material in combination with an antibacterial compound.

4. A structure according to Claim 3 wherein the odor-controlling agent is a member selected from the group consisting of carbon, silica gel, zeolite, siliceous molecular sieve, activated alumina, and mixtures thereof.

5. A structure according to Claim 4 wherein the front face film comprises a tapered capillary sheet.

6. An adult incontinence garment, absorbent pad, pantiliner, catamenial or diaper according to Claim 5.

7. A structure according to Claim 4 wherein the front face film comprises a formed-film sheet.

8. An adult incontinence garment, absorbent pad, pantiliner, catamenial or diaper according to Claim 7.

9. A structure according to Claim 1 wherein the odor-controlling agent is a siliceous molecular sieve having a pore diameter of at least 5.5 Angstroms.

**Fig.1**

200
201
2
2
202

**Fig.2**

200
201
202

**Fig.3**

278
234
226
224
256
210
239
239
256'
226'
212
234'
224'
278'
4
4

**Fig.4**

210
212
200
256'
239
234
224
230
226
256
214
226'
224'
230
234'
239
236
238
220
222
218
236'
238'